Europäisches Patentamt

European Patent Office

Office européen des brevets

(11) Veröffentlichungsnummer: **0 009 657**
**B1**

(19)

(12) # EUROPÄISCHE PATENTSCHRIFT

(45) Veröffentlichungstag der Patentschrift:
23.06.82

(21) Anmeldenummer: **79103328.5**

(22) Anmeldetag: **07.09.79**

(51) Int. Cl.³: **A 61 K 31/54** // (A61K31/54,
31/505)

(54) Mittel mit diuretischer Wirkung enthaltend 2,4-Diamino-5-(4-amino-3,5-dimethoxybenzyl)-pyrimidin und ein Kalium-ausschwemmendes Diuretikum und deren Herstellung.

(30) Priorität: **12.09.78 CH 9547/78**

(43) Veröffentlichungstag der Anmeldung:
**16.04.80 Patentblatt 80/8**

(45) Bekanntmachung des Hinweises auf die Patenterteilung:
**23.06.82 Patentblatt 82/25**

(84) Benannte Vertragsstaaten:
**AT BE CH DE FR GB IT LU NL SE**

(56) Entgegenhaltungen:
**DE-A-2 443 682**

Die Akte enthält technische Angaben, die nach dem Eingang der Anmeldung eingereicht wurden und die nicht in dieser Patentschrift enthalten sind.

(73) Patentinhaber: **F. HOFFMANN-LA ROCHE & CO.
Aktiengesellschaft, CH-4002 Basel (CH)**

(72) Erfinder: **Daum, Adam, Dr., Clarastrasse 45,
CH-4058 Basel (CH)**
Erfinder: **Fernex, Michel, Dr., Biederthal,
F-68450 Durmenach (FR)**
Erfinder: **Wick, Alexander Eduard, Dr., Rue du Buisson
Richard 10, F-78600 Le Mesnil-le Roy (FR)**

(74) Vertreter: **Lederer, Franz, Dr. et al, Patentanwälte Dr.
Franz Lederer Reiner F. Meyer Lucile-Grahn-Strasse 22,
D-8000 München 80 (DE)**

# Mittel mit diuretischer Wirkung, enthaltend 2,4-Diamino-5-(4-amino-3,5- dimethoxybenzyl)-pyrimidin und ein Kalium ausschwemmendes Diuretikum und deren Herstellung

Zu den heutzutage am häufigsten verwendeten Diuretica gehören die Gruppe der oral wirksamen Sulfonamide vom Thiazidtyp (Benzothiadiazinderivate), wie Hydrochlorothiazid und Hydrofluomethiazid, und Thiazidanaloga, wie Quinethazon, Chlorthalidon und Mefrusid. Diese Diuretica sind jedoch nicht frei von unerwünschten Nebenwirkungen, die auf eine erhöhte Kaliumausscheidung beim Patienten zurückzuführen sind und sich z. B. als Anorexie, Übelkeit, Schwäche oder Müdigkeit äußern können. Dieser Kaliumverlust ist besonders zu Beginn der Therapie ausgeprägt, vor allem wenn die Diurese stark ist und eine strikte Diät eingehalten wird. In der Regel wird dem Patienten zusätzlich ein Kaliumsalz, vorzugsweise Kaliumchlorid, verordnet, um den Kaliumverlust zu kompensieren. Andererseits sind bereits Kalium-retinierende Diuretica bekannt, die als Aldosteronantagonisten (Spironolacton) oder Pseudo-Aldosteronantagonisten (Triamteren) wirken und damit die Retention von Kalium verursachen. Spironolacton und das Triamteren werden vorwiegend in Kombination mit Thiaziden oder anderen Diuretica verabreicht (F. H. Meyers et al., Lehrbuch der Pharmakologie, Springer-Verlag, 1975, S. 178 – 189 und 768).

Es wurde nun überraschenderweise gefunden, daß auch 2,4-Diamino-5-(4-amino-3,5-dimethoxybenzyl)-pyrimidin eine ausgesprochene Kalium-retinierende Wirkung aufweist. Das gleiche gilt für seine physiologisch verträglichen Salze.

Von diesen Verbindungen wußte man bisher nur, daß sie antibakterielle Aktivität aufweisen und die Wirkung antibakteriell wirksamer Sulfonamide potenzieren (z. B. deutsche Offenlegungsschrift Nr. 2 443 682). Es hat sich gezeigt, daß die obengenannte Verbindung und ihre physiologisch verträglichen Salze gegenüber bereits bekannten Kalium-retinierenden Diuretica Vorteile besitzen und sie daher als Kalium-retinierende Mittel in Kombination mit Diuretica, die eine erhöhte Kaliumausscheidung verursachen, verwendet werden können.

Die vorliegende Erfindung betrifft daher Mittel (pharmazeutische Präparate) mit diuretischer Wirkung auf der Basis eines Kalium-ausschwemmenden Diureticums, die gekennzeichnet sind durch einen Gehalt an einem solchen Kalium-ausschwemmenden Diuretikum und an 2,4-Diamino-5-(4-amino-3,5-dimethoxybenzyl)-pyrimidin einen physiologisch verträglichen Salz davon sowie deren Herstellung.

Als physiologisch verträgliche Salze des 2,4-Diamino-5-(4-amino-3,5-dimethoxybenzyl)-pyrimidins kommen alle üblichen und dem Fachmann geläufigen in Frage, die mit organischen oder anorganischen Säuren wie Salzsäure, Schwefelsäure, Phosphorsäure, Ameisensäure, Essigsäure, Oxalsäure, Weinsäure, Maleinsäure, Benzoesäure, Bernsteinsäure, Fumarsäure, Lävulinsäure, Salicylsäure, Citronensäure, Isocitronensäure, Adipinsäure, Milchsäure, α-Ketoglutarsäure, Apfelsäure, Malonsäure, Glycerinsäure, Mevalonsäure, Gluconsäure, Neuraminsäure, Glutarsäure, Glucarsäure, Asparaginsäure, Gluconsäure, Mandelsäure, Ascorbinsäure, Lactobionsäure, Glucoheptonsäure, Glutaminsäure, Nicotinsäure, Pantothensäure, Folsäure, Adenylsäure, Geranylsäure, Cytidinsäure und Inosinsäure gebildet werden.

In den erfindungsgemäßen pharmazeutischen Präparaten kann die obengenannte Pyrimidinverbindung mit all jenen Diuretica kombiniert werden, die Kalium-ausschwemmend wirken. Dies sind vor allem stark wirksame Diuretica vom Thiazidtyp und Analoga, wie Hydrochlorothiazid, Hydrofluomethazid, Trichlormethiazid, Thiabuzid, Polythiazid, Cyclopenthiazid, Quinethazon, jedoch kommen auch nicht dem Thiazidtyp zugehörige Diuretica mit der genannten Nebenwirkung, wie z. B. Ethacrynsäure, in Betracht. Hydrochlorothiazid ist ein bevorzugter Kombinationspartner.

Die erfindungsgemäßen Präparate enthalten neben den Wirkstoffen — die direkt oder verzögert freigegeben werden können — die üblichen, für die orale oder parenterale Applikation geeigneten inerten organischen oder anorganischen Trägermaterialien, insbesondere die für Tabletten, Dragées, Kapseln oder Injektionslösungen üblichen sowie ggf. weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der jedem Fachmann geläufigen Weise erfolgen, durch Vermischen der aktiven Komponenten mit den geeigneten inerten organischen oder anorganischen Trägermaterialien und für die orale oder parenterale Verabreichung zweckmäßige galenische Formgebung.

Mit dem obengenannten Pyrimidin-Derivat ist eine sehr hohe, teilweise bis zu 90% gehende, maximale Kaliumretention erreichbar, was eine relativ geringe Tagesdosis erlaubt, die zweckmäßigerweise zwischen 1 und 20 mg, vorzugsweise bei 5 – 10 mg liegt. Ihre Dosierung in den pharmazeutischen Präparaten wird aber vor allem von der Höhe des durch die diuretisch wirkende Komponente verursachten Kaliumverlustes bestimmt. Pro Applikationseinheit kann sie ebenfalls 1 – 20 mg betragen. Wie bei jeder diuretischen Behandlung ist die Dosierung der Präparate dem Grad des Gewichtsverlustes und den Serumelektrolyttitern des Patienten anzupassen und durch den Arzt zu überwachen und festzusetzen. Die optimale Dosierung richtet sich nach dem Serumkaliumspiegel.

## Beispiel

Es wurden Tabletten der folgenden Zusammensetzung hergestellt:

| | |
|---|---|
| 2,4-Diamino-5-(4-amino-3,5-dimethoxybenzyl)-pyrimidin | 10,0 mg |
| Hydrochlorothiazid | 50,0 mg |
| D-Mannit | 43,0 mg |
| Maisstärke | 50,0 mg |
| Polyvinylpyrrolidon | 5,0 mg |
| Talk | 1,5 mg |
| Magnesiumstearat | 0,5 mg |
| | 160,0 mg |

Die Komponenten 1–3 und 80% der Komponente 4 wurden gesiebt und gemischt und mit einer ausreichenden Menge Feinsprit und Wasser sowie der Komponente 5 verarbeitet und granuliert. Das Granulat wurde getrocknet und gesiebt und nach Vermischen mit den restlichen Komponenten zu Tabletten mit Bruchrille à 160,0 mg verpreßt.

### Patentansprüche

1. Mittel mit diuretischer Wirkung auf der Basis eines Kalium-ausschwemmenden Diuretikums, gekennzeichnet durch einen Gehalt an einem solchen Kalium-ausschwemmenden Diuretikum und an 2,4-Diamino-5-(4-amino-3,5-dimethoxybenzyl)-pyrimidin oder einem physiologisch verträglichen Salz davon.

2. Mittel gemäß Anspruch 1, dadurch gekennzeichnet, daß das Kalium-ausschwemmende Diuretikum Hydrochlorothiazid ist.

3. Verfahren zur Herstellung von Mitteln gemäß den Ansprüchen 1 und 2, dadurch gekennzeichnet, daß man die Wirkstoffe, ggf. in Gegenwart üblicher inerter Träger- und Hilfsstoffe, miteinander vermischt und, gewünschtenfalls, das Gemisch in eine geeignete galenische Verabreichungsform bringt.

### Claims

1. Composition with diuretic activity based on a potassium-flushing diuretic, characterized by a content of such a potassium-flushing diuretic and of 2,4-diamino-5-(4-amino-3,5-dimethoxybenzyl)-pyrimidine or a physiologically compatible salt thereof.

2. Composition in accordance with claim 1, characterized in that the potassium-flushing diuretic is hydrochlorothiazide.

3. Process for the manufacture of compositions in accordance with claims 1 and 2, characterized by mixing the active substances with one another, optionally in the presence of customary inert carrier and adjuvant substances, and, if desired, bringing the mixture into a suitable galenical administration form.

### Revendications

1. Agent à action diurétique à base d'un diurétique hypokaliémique, caractérisé en ce qu'il contient un tel diurétique hypokaliémique et la 2,4-diamino-5-(4-amino-3,5-diméthoxybenzyl)-pyrimidine ou un de ses sels physiologiquement acceptables.

2. Agent selon la revendication 1, caractérisé en ce que le diurétique hypokaliémique est l'hydrochlorothiazide.

3. Procédé de préparation d'agents selon les revendications 1 et 2; caractérisé en ce qu'on mélange les substances actives, éventuellement en présence de supports et d'additifs inertes et, si on le désire, en ce qu'on amène le mélange sous forme d'administration galénique appropriée.